(19) Europäisches Patentamt

European Patent Office

Office européen des brevets

(11) EP 1 627 600 A1

(12) EUROPEAN PATENT APPLICATION

(43) Date of publication:
22.02.2006 Bulletin 2006/08

(51) Int Cl.:
$A61B\ 5/11^{(2006.01)}$   $A61B\ 5/0488^{(2006.01)}$

(21) Application number: 04447141.5

(22) Date of filing: 09.06.2004

(84) Designated Contracting States:
AT BE BG CH CY CZ DE DK EE ES FI FR GB GR HU IE IT LI LU MC NL PL PT RO SE SI SK TR
Designated Extension States:
AL HR LT LV MK

(71) Applicant: Université Libre de Bruxelles
1050 Brussels (BE)

(72) Inventors:
• Manto, Mario
B-1440 Braine-Le Château (BE)

• Pons Rovira, Jose
28007 Madrid (ES)
• Ceres Ruiz, Ramon
28007 Madrid (ES)
• Brunetti, Fernando
28007 Madrid (ES)

(74) Representative: Van Malderen, Joelle et al
pronovem - Office Van Malderen
Avenue Josse Goffin 158
1082 Bruxelles (BE)

(54) Portable medical device for automatic electrical coherence analysis inside a patient

(57) The present invention is related to a portable medical device for automatic monitoring of the electrical activity of target areas inside a patient, said medical device based on a wireless communication network comprising:

- a measuring unit (1) for measuring, as a function of a monitoring frequency, electrical signals at the surface of said two target areas (10,11);
- a processing unit (4) connected to the measuring unit (1), said processing unit (4) comprising:

(i) a slave unit (5) and a master unit (6) connected to said slave unit (5) for collecting and analysing the electrical signals measured by the measuring unit;
(ii) possibly a basic unit, and
(iii) a visualisation unit (7),

wherein the master unit (6) and the possibly basic unit (8) are configured so as to allow an analysis of the electrical signals comprising the analysis of the coherence between said first and second sets of electrical signals as a function of the monitoring frequency.

FIG. 2b

EP 1 627 600 A1

**Description**

**Field of the invention**

The present invention concerns the field of medical devices.
More precisely, the present invention is related to a new device for analysing physiological signals, and preferably tremors, inside a patient.

**State of the art**

Human tremor corresponds to a rhythmic activity of muscles and is the most common movement disorder. Recent studies have shown that 3 to 4% of the population aged higher than 60 years present a tremor in limbs and/or head.
Different medical devices have already been proposed in prior art for monitoring physiological signals inside a patient, and namely for monitoring electromyography signals. Examples of commercially available telemetry devices are MyoMonitor (EMG Systems), MT8 Telemetry system (MIE Medical Research Ltd) and MESPEC 4000 Telemetry (MEGA Electronics).
Tools for monitoring signals during daily life have also been proposed. For example, Keijsers N.L. et al. ("Online monitoring of dyskinesia in patients with parkinson's disease", *IEEE EMB Magazine,* vol.May/june, pp.96-103, 2003) have proposed accelerometers for online monitoring of dyskinesia.
Most of said devices use sensors for measuring tremor signals in the form of surface electrodes or needle electrodes to be placed at specific target areas on the patient, including brain and muscles.
However, said medical devices present several drawbacks which limit their use. Indeed, said devices do not automatically provide relevant informations about the signals measured which could be instantaneously used as such by a doctor for a diagnosis. On the contrary, the data collected by said devices require further investigations under the control of very specialised people trained therefore in dedicated centers. In addition, the size of said devices is such that a signal monitoring at the bed of the patient (bedside examination) is impossible and the displacement of the patient is necessary.
In other words, the prior art devices provide a signal monitoring which is time-consuming, expensive, and uncomfortable for the patient.
There is thus a rather important need for an enhanced medical device, and namely a medical device adapted to tremor analysis.

**Aims of the invention**

The present invention aims to provide a medical device for monitoring physiological signals in a patient, and especially tremor signals, which does not present the drawbacks of the prior art devices as disclosed hereabove.
In particular, the present invention aims to provide a portable medical device which is easy to use, of moderate cost and which provides automatically, and instantaneously if required, a relevant analysis of the measured signals which could be used directly by a doctor for a diagnostic.
Another aim of the present invention is to provide a medical device which is non invasive.

**Summary of the invention**

The present invention is related to a portable medical device for automatic monitoring of the electrical activity of target areas inside a patient, said medical device comprising:

- a measuring unit comprising at least a first sensor and a second sensor for measuring, as a function of a monitoring frequency, a first set of electrical signals at the surface of a first patient target area, and a second set of electrical signals at the surface of a second patient target area, respectively;
- a processing unit connected to the measuring unit, said processing unit comprising:

   (i) a slave unit for collecting the first set of electrical signals measured by the measuring unit;
   (ii) a master unit connected to said slave unit for receiving the first set of electrical signals collected by the slave unit, for collecting the second set of electrical signals measured by the second sensor (and for analysing the first set and the second set of electrical signals thus collected);
   (iii) a visualisation unit, such as a LCD display, connected to the master unit for at least presenting the result of the electrical signals analysis performed by the master unit, and
   (iv) possibly a basic unit connected to the master unit for receiving and recording the first set and the second set of electrical signals collected by the master unit (analysing them and presenting the result of the obtained analysis under appropriate form for the clinician),

wherein the master unit and the possible basic unit are configured so as to allow an analysis of the electrical signals comprising the analysis of the coherence between said first and second sets of electrical signals as a function of the monitoring frequency, and wherein the device further comprises a wireless communication unit for ensuring a wireless communication connection between the different units of the device.

Preferably, the electrical signals correspond to tremor signals.

Advantageously, the sensors are selected from the group consisting of electromyography sensors, electro-encephalography sensors and/or cortical sensors.

Preferably, said sensors are biomechanical sensor electrodes, such as surface electrodes, (intra-muscular) needle electrodes or micro-electrodes.

Advantageously, the present medical device is adapted for inter-muscular or intra-muscular tremor coherence analysis.

Preferably, said medical device is adapted for working at monitoring frequencies comprised between about 0,1 Hz and about 30Hz, preferably between about 3Hz and about 16Hz, and more preferably between about 3Hz and about 12Hz.

Preferably, the basic unit comprises a configuration tool to set up the configuration parameters of the device in operating conditions, said configuration parameters comprising the sampling frequency at which the master and slave units have to work and the monitoring frequencies.

Advantageously, the wireless communication unit of the present medical device uses the Bluetooth technology.

The present invention also concerns the use of the present medical device as a tool for characterising tremors related to specific diseases.

Advantageously, the present medical device may also be used as a tool for testing the effect of drugs on tremors.

However, it has to be noted that the medical device according to the present invention provides physical data corresponding to intermediate results concerning the tremor which do not on their own enable a decision to be made by the doctor or surgeon on a diagnostic or treatment.

## Short description of the drawings

Fig. 1 represents a schematic view of the different units of a device according to the present invention.

Fig. 2a shows an example of application of said device for monitoring intralimb tremor coherence.

Fig. 2b-2c and Fig.3 show an example of application of said device for monitoring interlimb tremor coherence.

Fig.4 gives an example of EMG signals as acquired with one embodiment of a medical device according to the invention.

## Detailed description of the invention

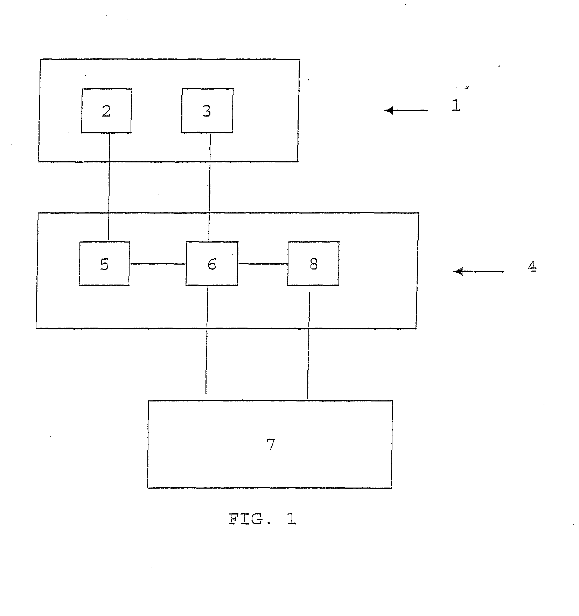

Fig.1 represents a diagram, wherein the different elements of a portable device according to the invention and their relationship with one another are represented.

As shown in Fig.1, said medical device comprises a measuring unit 1 comprising at least a first sensor 2 and a second sensor 3. Said sensors 2,3 may be electromyography sensors, electro-encephalography sensors, or cortical sensors, and preferably take the form of electrodes possibly connected to a battery (15) and combined with low-pass or high-pass filters. Said electrodes may be for example surface electrodes or (intramuscular) needle electrodes possibly bound to the skin 12 by an interface adhesive 13 or inserted in the muscles 14 (at specific locations 10,11).

Furthermore, the device may comprise electromyographic amplifiers that advantageously improve the signal/ noise ratio.

Both first and second sensors 2,3 are able in operating conditions to measure electrical activities at monitoring frequencies comprised between about 0,1 Hz and about 30Hz, preferably between about 3Hz and about 16Hz, and more preferably between about 3Hz and about 12Hz.

The medical device according to the invention also comprises a processing unit 4 connected to the measuring unit 1 by means of a wireless communication network using, for example, the Bluetooth technology or the HomeRF or the WiFi technology or the IrDA technology.

The function of the processing unit 4 is to process (amplification, filtration, analysis) in operating conditions the data concerning the electrical signals measured and transmitted by the sensors 2,3 in such a way as to provide relevant information directly available to a clinician (obtained coherence value is computed, stored and displayed) but it can also be monitored and further analysed off-line by means of additional displaying hardware and software.

More precisely, said processing unit 4 comprises a slave unit 5 which is connected to the first sensor 2 for collecting the first set of electrical signals measured by said first sensor 2.

Said slave unit 5 is connected to a master unit 6 in such a way that the master unit 6 may receive in operating conditions the first set of electrical signals collected by the slave unit 4.

The master unit 6 is also connected to the second sensor 3 for collecting the second set of electrical signals measured by said second sensor 3.

In addition, the master unit 6 is configured so as to allow an analysis of the first set and the second set of electrical signals it has thus collected.

A basic unit 8 is optionally connected to the master unit 6 for receiving and recording the first set and the second set of electrical signals collected by the master unit 6, analysing them and presenting the result of said analysis under appropriate form for the clinician.

In other words, both the master unit 6 and the basic unit 8 are able to perform the analysis of the first set and the second set of electrical signals collected by the master unit 6.

The medical device further comprises a visualisation unit 7, such as a LCD display (8 columnsx2 lines character display), connected to the master unit 6 and possibly to the basic unit 8 which is able on demand to present the result of the electrical signals analysis performed by the master unit 6.

In the medical device according to the present invention, the master unit 6 and possibly the basic unit 8 are configured so as to allow an analysis of the electrical signals comprising the analysis of a coherence between the first and second sets of electrical signals as a function of a monitoring frequency.

The processing of the two sets of electrical signals by the master unit 6 and the basic unit 8 comprises the calculation of the function of coherence obtained by a configuration of these units (6 and 8) that apply the following coherence algorithm. Coherence is a bounded measure of linear correlation and is defined as follows for two signals x and y:

$$\gamma_{xy}(f) = \frac{G_{xy}}{\sqrt{G_{xx}(f)G_{yy}(f)}} \qquad (1)$$

wherein f denotes the considered frequency and $G_{xy}$ the complex cross power spectrum stated by :

$$G_{xy}(f) = \int_{-\infty}^{+\infty} R_{xy}(\tau)e^{j2\pi f\tau}d\tau, \qquad (2)$$

and is the Fourier transform of the cross correlation function:

$$R_{xy}(\tau)=E[x(t)y(t+\tau)].$$

wherein x and y (EMG signals) are real and E [] denotes the mathematical expectation.

Coherence is equal to 1 when one signal is a linear function of the second signal, and coherence is equal to 0 in the case of linear independence.

It should be noted that the dimensions, compositions and weight of the different units (elements) of the present medical device are such that it is portable and it can easily be placed in the pocket of clothes.

The device may also comprise one or more (electromyographic) amplifiers for amplifying adequately the acquired signals and improving signal/noise ratio.

The communication network between the different elements of the device is of wireless type, and uses, for example, the Bluetooth technology or the HomeRF or the WiFi technology or the IrDA technology.

As a consequence, the medical device has the advantage of being independent from any electrical installation and could be used in extreme conditions, as for example for quick examination of road accident victims.

As another consequence, the medical device could be easily used during bedside examination of patients, thereby being more comfortable for the patient than the prior art devices.

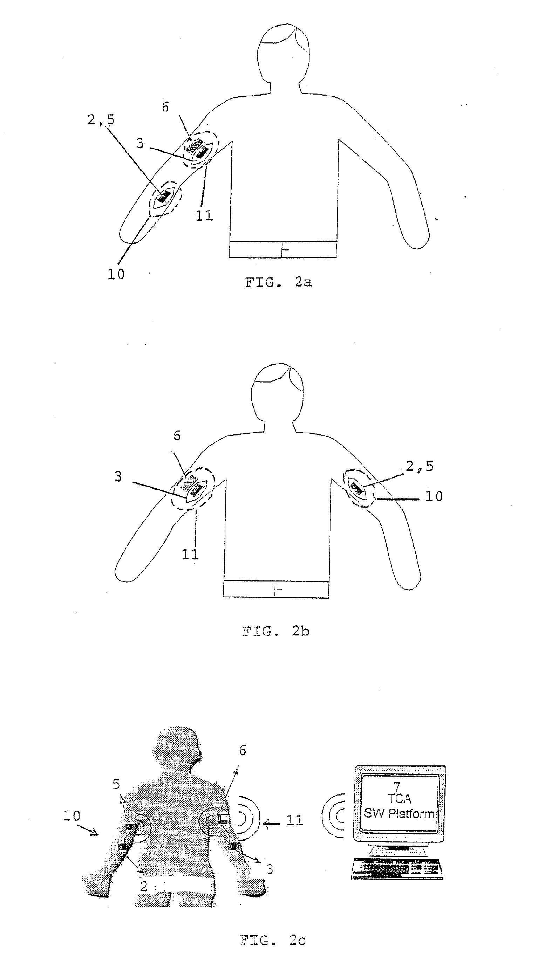

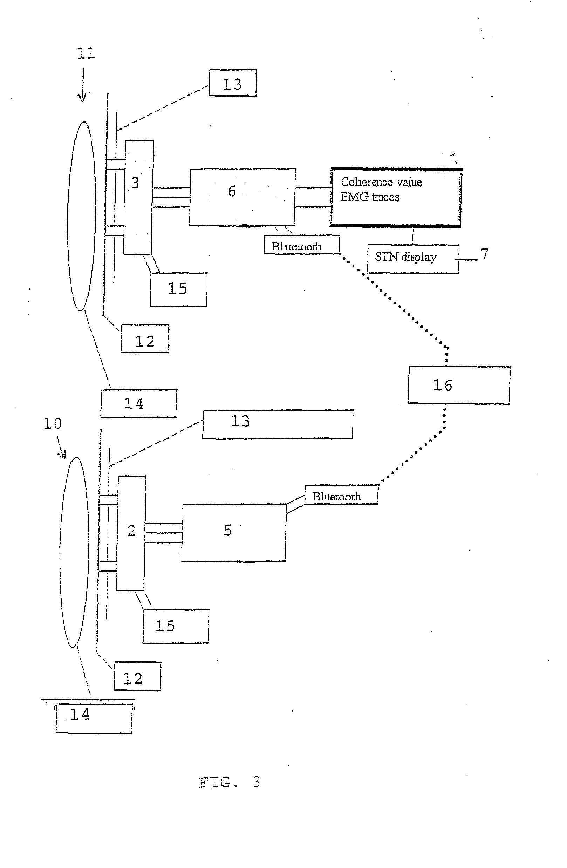

Fig.2a, Fig2b-2c and Fig.3 illustrate two examples of possible application of such medical device. As shown in Fig.2a, the sensors could be placed on two different areas 10,11 located on the same upper limb of a patient for monitoring intralimb muscular tremor coherence. The sensors 2, 3 may also be placed on two different areas 10,11 located each on a different upper limb of a patient for monitoring interlimb muscular tremor coherence, as illustrated in Fig.2b-2c and Fig.3.

However, it is important to note that the present medical device is not limited to said illustrations. Other applications of the medical device according to the invention could be envisaged by the person skilled in the art.

For example, the sensors may be adapted for a fixation on other patient target areas such as other muscles or brain.

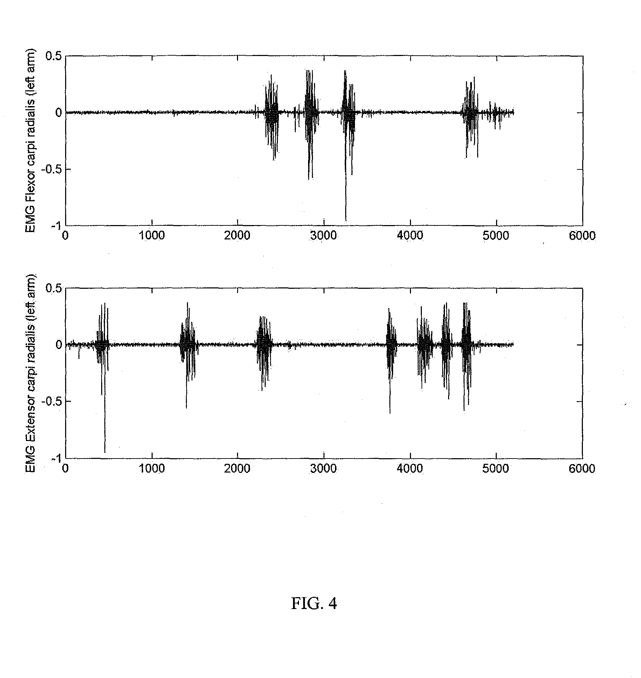

Fig.4 gives an example of EMG signals as acquired with one embodiment of a medical device according to the invention. The technical characteristics of the medical device in said embodiment were the following:

- master unit:

  - 3.6 Lithium Ion rechargeable battery
  - Microcontroller 3.3V
  - 3.3V voltage regulator
  - Signal Conditioning block.
  - Communication with the Bluetooth module using serial port at 115kbps
  - 7.32 MHz system clock.
  - Sampling Frequency: 512 Hz.

- slave unit: idem as for the master unit
- signal conditioning module (in both main and slave units) :

  - Signal Amplification.
  - Low pass filter (400 Hz).
  - Signal Rectification.
  - Diode clipping circuit.
  -

- Computer (basic unit 8):

  - Windows operating system.
  - Developed TCA software.
  - RS232 - Serial port.
  - Bluetooth module.

Beside muscular tremor coherence analysis, the medical device may also be configured in such a as to allow a monitoring of coherence between other physiological signals detectable through electrical activity measurement, such as cortico-cortical coherence analysis and cortico-muscular coherence analysis.

Furthermore, the medical device could be used not only as a routine tool usable by a doctor during its daily practice, but also as a research tool for different investigations.

For example, the medical device could be used as a tool to investigate in a patient if there is a unique generator that generates tremors or if there are several tremor generators interacting with each other. From that point of view, the medical device could be considered as a useful tool providing data which may help doctors or clinicians to establish a better diagnostic (analysis of the electromyographic signals in real time with a direct display of the coherence value).

The present medical device could also be used by clinicians or pharmaceutical companies to test the effects of drugs or other kinds of stimuli on the electrical activity (changes, events, actions) by adirect assessment of the effect of said drugs and stimuli on the coherence between electromyographic signals.

The present medical device finds also useful applications such as:

- assessment of deep brain stimulation (stimulation of thalamic nuclei Vim, stimulation of subthalamic nuclei STN) on the coherence;
- on line direct estimation of the value of intermuscular coherence in orthostatic tremor;
- detection of the shift from a low coherence to a high coherence, as observed in high-frequency synchronous discharges;
- epidemiological analysis of intermuscular coherence in Parkinson's disease and essential tremor;
- investigations of the effects of extra-load on the coherence in rest, postural and kinetic tremor;

- investigations of the effects of fatigue or vibrations on the coherence between muscles.
- estimation of coherence between two or more muscle fibers from one or different muscles, whose discharges are preferably recorded using micro-electrodes.

**Claims**

1. A portable medical device for automatic monitoring of the electrical activity of target areas (10,11) inside a patient, said medical device comprising:

   - a measuring unit (1) comprising at least a first sensor (2) and a second sensor (3) for measuring, as a function of a monitoring frequency, a first set of electrical signals at the surface of a first patient target area (10), and a second set of electrical signals at the surface of a second patient target area (11), respectively;
   - a processing unit (4) connected to the measuring unit, said processing unit (4) comprising:

     (i) a slave unit (5) for collecting the first set of electrical signals measured by the measuring unit;
     (ii) a master unit (6) connected to said slave unit (5) for receiving the first set of electrical signals collected by the slave unit (5), for collecting the second set of electrical signals measured by the second sensor (3);
     (iii) a visualisation unit (7), connected to the master unit for at least presenting the result of the electrical signals analysis performed by the master unit (6);
     (iv) and possibly a basic unit (8) connected to the master unit (6) for receiving and recording the first set and the second set of electrical signals collected by the master unit (6), analysing them and presenting the result of said analysis under appropriate form for the clinician,

   wherein the master unit (6) and the possibly basic unit (8) are configured so as to allow an analysis of the electrical signals comprising the analysis of the coherence between said first and second sets of electrical signals as a function of the monitoring frequency, and wherein the device further comprises a wireless communication unit (16) for ensuring a wireless communication connection between the different units of the device.

2. The medical device according to claim 1, **characterised in that** the electrical signals correspond to tremor signals.

3. The medical device according to claim 1 or 2, **characterised in that** the sensors (2,3) are selected from the group consisting of electromyography sensors, electro-encephalography sensors and/or cortical sensors.

4. The medical device according to any one of the preceding claims, **characterised in that** the sensors (2,3) are biomechanical sensor electrodes.

5. The medical device according to any one of the preceding claims, **characterised in that** it is adapted for inter-muscular tremor coherence analysis.

6. The medical device according to any one of the preceding claims, **characterised in that** it is adapted for working at monitoring frequencies comprised between about 0,1Hz and about 30Hz, preferably between about 3Hz and about 16Hz, and more preferably between about 3Hz and about 12Hz.

7. The medical device according to any one of the preceding claims, **characterised in that** the basic unit (8) comprises a configuration tool to set up the configuration parameters of the device in operating conditions, said configuration parameters comprising the sampling frequency at which the master and slave units have to work and the monitoring frequencies.

8. The medical device according to claim 1, **characterised in that** the wireless communication unit (16) uses the Bluetooth technology.

9. The medical device according to any one of the preceding claims, wherein the visualisation unit (7) is a LCD display.

10. The medical device of claim 4, wherein the electrodes are selected from the group consisting of surface electrodes, needle electrodes or micro-electrodes.

FIG. 1

FIG. 2a

FIG. 2b

FIG. 2c

FIG. 3

FIG. 4

European Patent
Office

**EUROPEAN SEARCH REPORT**

Application Number

EP 04 44 7141

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | US 2001/041846 A1 (DAY MARY KATHLEEN ET AL) 15 November 2001 (2001-11-15) * paragraphs [0041] - [0055] * ----- | 1-10 | A61B5/11 A61B5/0488 |
| A | US 6 597 944 B1 (HADAS NOAM) 22 July 2003 (2003-07-22) * column 5, line 30 - column 8, line 54 * ----- | 1-10 | |
| A | US 5 265 619 A (BURGER GUY-CLAUDE ET AL) 30 November 1993 (1993-11-30) * column 3, line 17 - column 7, line 23 * * claims 1-23 * ----- | 1-10 | |
| A | US 5 505 208 A (KASTEN JOHN D ET AL) 9 April 1996 (1996-04-09) * column 4, line 10 - column 6, line 50 * ----- | 1 | |

TECHNICAL FIELDS
SEARCHED (Int.Cl.7)

A61B

The present search report has been drawn up for all claims

| Place of search | Date of completion of the search | Examiner |
|---|---|---|
| Munich | 18 October 2004 | Rivera Pons, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 1 627 600 A1**

**ANNEX TO THE EUROPEAN SEARCH REPORT
ON EUROPEAN PATENT APPLICATION NO.**

EP 04 44 7141

This annex lists the patent family members relating to the patent documents cited in  the above-mentioned European search report.
The members are as contained in the European Patent Office EDP file on
The European Patent Office is in no way liable for these particulars which are merely  given for the purpose of information.

18-10-2004

| Patent document cited in search report | | | Publication date | Patent family member(s) | | | Publication date |
|---|---|---|---|---|---|---|---|
| US 2001041846 | A1 | | 15-11-2001 | US | 6280395 | B1 | 28-08-2001 |
| | | | | AU | 2961601 | A | 31-07-2001 |
| | | | | WO | 0152732 | A1 | 26-07-2001 |
| US 6597944 | B1 | | 22-07-2003 | AU | 2823800 | A | 21-09-2000 |
| | | | | CA | 2362019 | A1 | 08-09-2000 |
| | | | | EP | 1164926 | A2 | 02-01-2002 |
| | | | | WO | 0051543 | A2 | 08-09-2000 |
| | | | | JP | 2003524459 | T | 19-08-2003 |
| US 5265619 | A | | 30-11-1993 | FR | 2645641 | A1 | 12-10-1990 |
| | | | | AT | 86383 | T | 15-03-1993 |
| | | | | CA | 2048688 | A1 | 11-10-1990 |
| | | | | DE | 69001019 | D1 | 08-04-1993 |
| | | | | DE | 69001019 | T2 | 26-08-1993 |
| | | | | EP | 0467980 | A1 | 29-01-1992 |
| | | | | WO | 9012293 | A1 | 18-10-1990 |
| | | | | JP | 4504519 | T | 13-08-1992 |
| | | | | JP | 3234595 | B2 | 04-12-2001 |
| US 5505208 | A | | 09-04-1996 | NONE | | | |

EPO FORM P0459

For more details about this annex : see Official Journal of the European Patent Office, No. 12/82